# EUROPEAN PATENT APPLICATION

(11) **EP 2 478 926 A1**
(43) Date of publication of application: **25.07.2012**
(21) Application number: 11151566.4
(22) Date of filing: 20.01.2011
(51) Int. Cl.: A61M 16/06, A44B 11/25, A44B 11/26

(54) **Connector element for headgear of respiratory mask**

(71) Applicant: Air Liquide Medical Systems, 92160 Antony (FR)
(72) Inventor: Alberici, Luca, 25128, Brescia (IT); Rivetti, Alberto, Rodengo Saiano (BS) 25050, Rovato (BS) (IT); Sandoni, Giuseppe, 25124, Brescia (IT)
(74) Representative: Pittis, Olivier

(57) **Abstract**

A connector element (11) for a respiratory mask, especially a facial mask, comprising a front portion (11a) of a third axis (CC) facing a rear portion (11b), and two lateral arms (12a, 12b) linking together said front and rear portions so as to form a frame (11a, 11b, 12a, 12 b). The frame (11a, 11b, 12a, 12b) bears a tongue element (15) made of a resilient material so as to be deformable, bendable or pivotable with respect to the third axis (CC), when a force is applied on the upper surface (15a) of said tongue element (15).Further, one or several first coupling structures (14) are provided on the upper surface (15a) of the tongue element (15). The lateral arms (12a, 12b) each have respectively a first axis (AA) and a second axis (BB) forming an angle (α) of between 95° and 150° with the third axis (CC). A respiratory mask (1) comprising a headgear (9) with one or more connector elements (11) according to the present invention is suitable for the treatment of respiratory disorders, such as obstructive sleep apnea (OSA).

## Description

The invention concerns a connector element for a respiratory mask, in particular, a facial mask for use in the treatment of respiratory conditions or diseases such as obstructive sleep apnea, and an assembly comprising a respiratory mask connected to said connector element which is itself connected to the headgear of the mask.

Masks, such as facial or nasal masks, are commonly used for delivering non-invasive positive pressure ventilation (NPPV) or for nasal continuous positive airway pressure (N-CPAP) therapy in sleep disordered breathing (SDB) conditions such as obstructive sleep apnea (OSA). These masks typically deliver a flow of breathable gas for, or to assist in, patient respiration, especially during the night, i.e., when the patient is sleeping.

Such a mask assembly typically comprises a rigid or semi-rigid hollow shell, also called a mask body, defining a breathing chamber that receives at least a part of the patient's nose and/or mouth, and further comprising a soft face-contacting cushion that comes into contact with the patient's face, a forehead support that is optionally pivotable and a headgear for correctly positioning, maintaining and/or securing the mask on the head of a patient.

The shell of the mask or mask body is connected to a gas supply line which delivers a respiratory gas, such as air under pressure, into the breathing chamber of the shell through a gas opening or inlet orifice arranged in the wall of the mask body. The connection between the gas line and the mask body is usually achieved by means of a hollow connector comprising an internal passage for the gas. The connector can have an elbow-shape, i.e. can be of a curved shape or any other shape. A rotatable connector that rotates around the axis of the inlet orifice is known to be used to correctly position the gas line, which is typically a flexible conduit, with respect to the mask.

The soft face-contacting cushion often comprises two superimposed membranes, e.g., an inner membrane covered by an outer membrane, that ensures the air tightness all around the periphery of the cushion in contact with the user's face.

These types of respiratory masks are known from documents such as EP-A-1737524, EP-A-1841481, EP-A-956069 or EP-A-1118346.

More precisely, when the mask is used by the patient, the outer membrane comes into contact with the different regions of the user's face, i.e., the nasal bridge region, the chin region and the two lateral cheek regions that join the nasal bridge region to the chin region. As both membranes are made of a resilient flexible or soft material, the membranes are deformed under the traction forces exerted by the headgear of the mask in order to match the profile and forms of the user's face, thereby ensuring the air tightness of the mask.

However, the existing masks equipped with headgear are not totally satisfying. Indeed, the problem that exists is that connecting the straps of the headgear to the mask body is often not easy for the user, who because of the structure of the mask and the positioning necessary to put the mask into use is not able to see the site of connection while making the connection.

Hence, the problem to be solved is to provide an improved connector element for respiratory masks, especially for facial masks, which allows an easy connection of the headgear to the mask body.

In other words, the problem is to propose a connector element allowing a quick coupling and a quick release of the headgear, and further having a structure that is as simple as possible, as well as a respiratory mask, especially a facial mask, equipped with such a connector element.

The solution provided by the present invention concerns a connector element comprising :
- a front portion of a third axis (CC) facing a rear portion, and two lateral arms linking together said front and rear portions so as to form a frame,
- the frame bearing a tongue element having an upper surface, said tongue element being made of a resilient material so as to be deformable, bendable or pivotable with respect to the third axis (CC) when a force is applied on the upper surface of said tongue element,
- the upper surface of said tongue element bearing one or several first coupling structures, and
- the two lateral arms of said frame having respectively a first axis (AA) and a second axis (BB), said first axis (AA) and said second axis (BB) each forming an angle (α) of between 95° and 150° with said third axis (CC).

The connector element for respiratory mask according to the present invention can further comprise one or more of the following additional features:
- the frame and the tongue element are molded in one-piece.
- the frame and the tongue element are made of a polymer material, such as polycarbonate (PC), polypropylene (PP), Acrylonitrile Butadiene Styrene (ABS), nylon or polystyrene (PS).
- the first axis (AA) and the second axis (BB) each form an angle (α) of between 100° and 140° with regard to the third axis (CC), preferably of between 105° and 130°.
- one or several first coupling structures are nipples or similar structures.
- the tongue element has a general flat-shape.
- the tongue element comprises an actuating zone arranged at the free end of said tongue element.
- the tongue element projects toward the center of the frame.
- the tongue element is arranged on the front portion of the third axis (CC) of the frame.
- the front portion of the third axis (CC) and a first portion of each of the two lateral arms respectively form the more narrow base and the two sides of a trapezoidal or quasi-trapezoidal structure.
- the rear portion and a second portion of each of the two lateral arms form three sides of a rectangular or quasi-rectangular structure.

The invention also concerns a respiratory mask, in particular a facial mask, comprising a mask body and at least one pocket arranged in the external wall of said mask body, said or each pocket being sized and shaped so as to receive a connector element according to the present invention, said or each pocket comprising at least one second coupling structure cooperating with at least one coupling structure of the tongue element of the connector element for maintaining said connector element in a locked position while inserted into said one pocket of the mask body.

The mask according to the present invention can further comprise one or more of the following additional features:
- the first coupling structures arranged on the tongue element are chosen from among various types of nipples and abutments, and the second coupling structures are chosen from among various types of lodgings and grooves.
- the mask further comprises a headgear with one or more straps, at least one of said straps being attached to the rear portion of the connector element.
- the mask further comprises a forehead support, an internal chamber and a gas inlet orifice that is in fluid communication with said internal chamber, and the internal chamber comprises a peripheral border bearing a cushion.
- the mask further comprises a cushion having a triangular or a saddle shape.
- the cushion comprises an inner membrane and an outer membrane.
- the cushion frame and the inner and outer membranes are molded into one piece and are preferably made of silicone.
- the outer membrane comprises a free curved edge projecting toward the interior of the cushion.
- the mask is a facial mask covering the nose and the mouth of the user.
- the mask further comprises a hollow gas-connector having an internal gas passage, such as an elbow-shaped connector, that is connected to a gas inlet orifice so as to be in fluid communication with the internal chamber of the mask.
- the hollow connector comprises an anti-asphyxia valve.

An embodiment of a connector element for a facial mask according to the present invention as well as a facial mask connected to such a connector element is shown in the enclosed Figures, among which :
- Figure 1 represents a facial mask according to the present invention when positioned on the patient's face,
- Figure 2 shows a connector element according to the present invention inserted in the mask body,
- Figure 3 represents the connector element according to the present invention when withdrawn from the mask body, and
- Figure 4 is a schematic representation of the connector element according to the present invention.

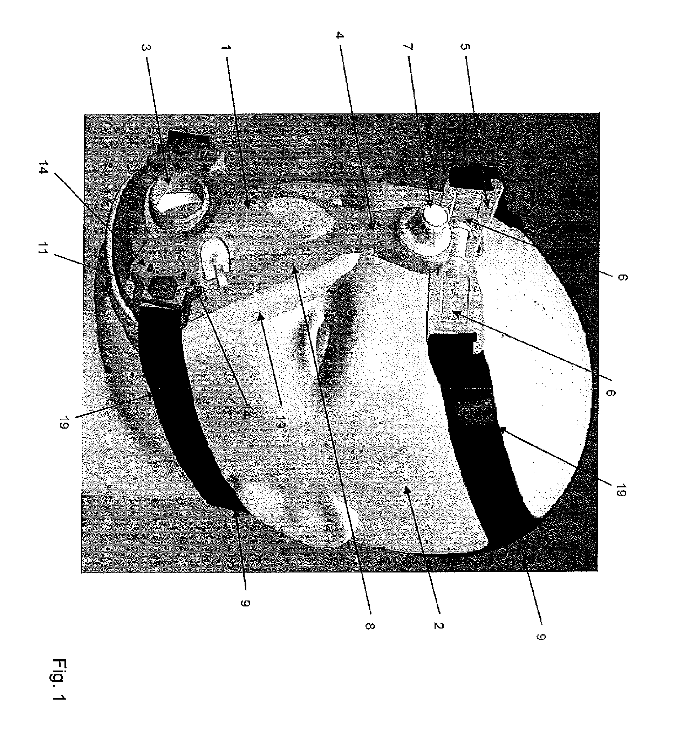

As illustrated in Figure 1, a respiratory facial mask according to the present invention comprises a rigid or semi-rigid hollow shell or mask body 1 that defines an internal breathing chamber or volume wherein respiratory gas, such as air under pressure, is introduced via an inlet port 3 which is connected to a gas feeding line, such as a flexible gas conduit, by means of a tubular hollow connector. Preferably, the hollow connector has a general elbow-shape and comprises an internal passage for the gas.

The gas inlet orifice 3 is arranged at the center of the mask body 1 and through the wall of the mask body 1 thereby allowing air under pressure to be introduced into the breathing chamber that receives the nose and mouth of the patient 2.

The mask body 1 is preferably made of a polymer material, such as polycarbonate (PC), polypropylene (PP), acrylonitrile butadiene styrene (ABS), nylon or polystyrene (PS), and is configured so as to be able to receive at least a part of the patient's nose and mouth. In other words, the patient introduces his/her nose and mouth into the internal volume of the breathing chamber of the mask body 1 and breathes the pressurized gas contained therein. The mask body 1 has preferably a general triangular or quasi-triangular three-dimensional shape as demonstrated in Figure 1 for example.

The mask body 1 further comprises an expansion part 4 arranged on the top of the mask body 1 and projecting upwardly from said mask body 1, i.e. projecting away from the external surface of the mask body 1. The expansion part 4 and the mask body 1 are made of polymeric material that is molded into one piece so as to obtain an expansion part 4 that is integral with the rest of the mask body 1.

Further, as represented in Figure 1, a forehead support 5 is connected to the mask body 1 by means of a pivotable holding arm fixed to or carried by either the mask body 1 or the expansion part 4, preferably by the expansion part 4. The forehead support 5 can comprise one or more pillows 6 of soft and comfortable material that come into contact with the patient's forehead. An acting piece (not visible in Figure 1) is arranged in a traversing orifice of the expansion part 4 and is mobile, preferably in translation, in said traversing orifice so as to cooperate with the holding arm for pivoting said holding arm, when said acting piece moves in the traversing orifice of the expansion part 4. Further, the forehead support 5 can also pivot with respect to the holding arm 4. Actually, the back or forward motion of the acting piece in the traversing orifice is obtained by manual rotation by the user of a rotating knob 7 cooperating with the acting piece when said rotating knob 7 is manually operated, i.e. turned clockwise or counterclockwise, by the user. The maximum course of the knob 6 is about 360° or less.

Furthermore, in order to ensure a tight positioning of the mask on the patient's face and to increase the comfort for the patient 2, the peripheral border or edge 8 of the mask body 1 comprises a cushion 10 made of soft, resilient, elastomeric material that comes into contact with the patient's face. Said cushion 10 has a central aperture for receiving the patient's nose and mouth. The central aperture of the cushion 10 is positioned with regard to the internal chamber of the mask body 1, i.e. they are facing each other. The cushion 10 arranged on the peripheral border 8 of the internal chamber of the mask body 1 comprises two superimposed flexible membranes ensuring an air tightness to minimize air leaks. Such types of cushions 10 and mask body 1 structures are well-known in the art and taught by many documents, such as EP-A-1334742, EP-A-264772, EP-A-956069, EP-A-874667, US 2,931,356 or EP-A-1479406. More precisely, the border 8 and the cushion 10 have a general triangular or saddle-shape structure so as to match the contours of the nasal region including the upper nasal bridge region, the cheek regions on both sides of the nose, and the lower chin region of the patient 2.

The shell or mask body 1 is fluidly linked to a gas supply line, such as a flexible hose or conduit that conveys respiratory gas to the mask, by means of a tubular hollow connector which delivers a respiratory gas, such as air under pressure, into the breathing chamber of the shell 1 through the gas inlet 3 arranged in the mask body 1. Gas under pressure, such as air, can be produced and delivered in a BPAP-type (bi-level positive airway pressure) or CPAP-type (continuous positive airway pressure) apparatus.

Further, a headgear 9 comprising straps 19 can be connected or disconnected to the mask body 1, by means of one or several connector elements 11 thereby maintaining the mask in a desired position on the patient's face during the use of the mask, thus allowing for an efficient treatment of sleep disorders, such as OSA or similar disorders.

Each connector element 11 is inserted in a pocket 30 arranged in or forming part of the external wall of the mask body 1. Generally, two pockets 30 are provided, one being arranged on each lateral side of the mask body 1 with respect to the central gas inlet or orifice 3, i.e., a right pocket 30 and a left pocket 30, each of them receiving a connector element 11.

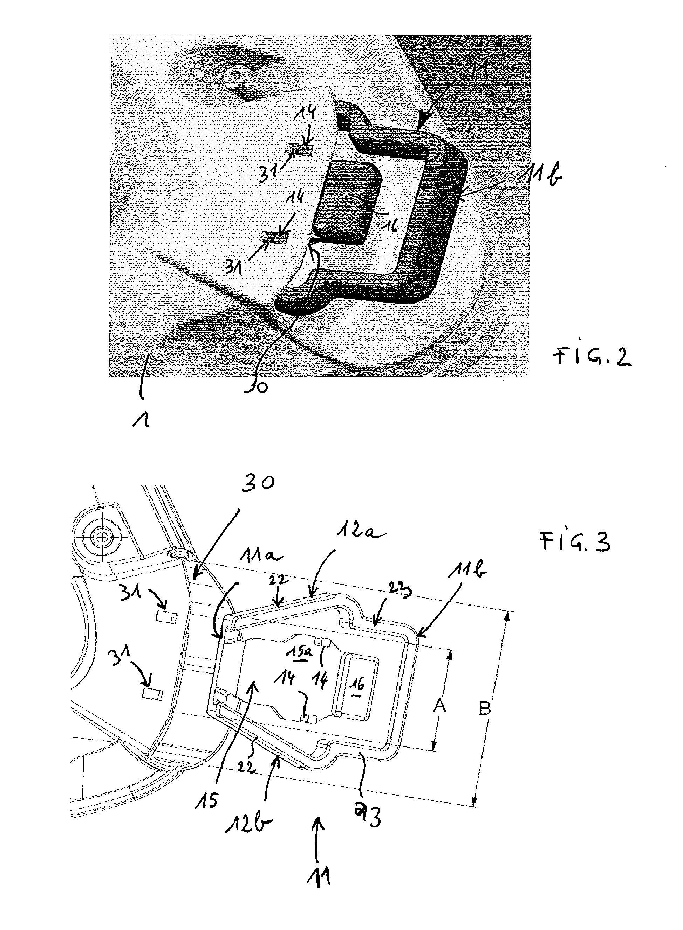

The pockets 30, as shown in Figures 2 and 3, are conformed and sized so as to have an internal shape that fits with the general shape of the connector element 11. In other words, the internal shape of each of the pockets 30 matches the shape of the connector element 11.

To avoid the patient having to open and close the Velcro straps 19 of the headgear 9 to put the mask in the right position on his/her face or in contrast, to remove it from his/her face, a connector element 11 comprising a quick coupling/releasing mechanism as detailed below is provided. This connector element 11 has a particular shape that allow for the quick insertion into or withdrawal of the connector element 11 from pockets 30 arranged on the mask.

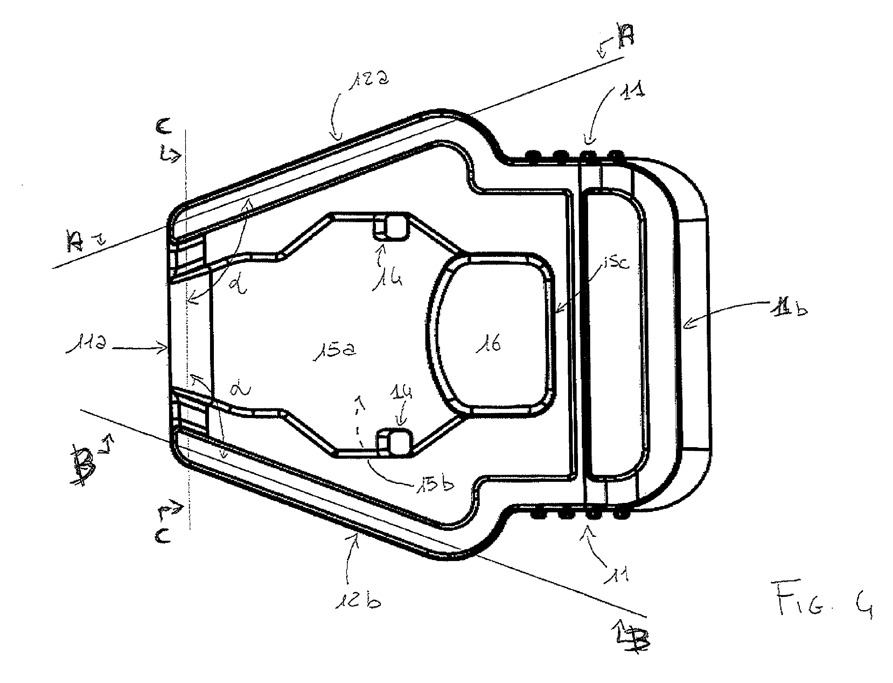

The structure of the connector element 11 according to the present invention is shown in Figures 2 to 4. The coupling/releasing mechanism of the present invention, i.e. the connector element 11, is simple but really effective. Thus, the connector element 11 of the present invention comprises a front portion 11a of the third axis (CC) facing a rear portion 11b, and two lateral arms 12a, 12b linking together said front and rear portions so as to form a frame 11a, 11b, 12a, 12 b, i.e. a kind of buckle or close loop. The front portion 11a and the rear portion 11b are preferably linear portions that are parallel to each other as shown in Figures 3 and 4. For example, the front portion 11a and the rear portion 11b can have a length of between 5 mm and 50 mm.

Further, the frame 11a, 11b, 12a, 12 b, such as the front portion 11a of third axis (CC) as shown in Figures 3 and 4, bears a tongue element 15, such as a plate-like structure, having an upper surface 15a and a lower surface 15b. The tongue element 15 is made of a resilient material, such as a plastic material, so as to be deformable, bendable or pivotable with respect to the frame 11a, 11b, 12a, 12 b, preferably with respect to the third axis (CC), when a force is applied by the user on the upper surface 15a, preferably on the actuating zone 16 situated at the free end 15c of the tongue element 15.

Further, the upper surface 15a of the tongue element 15 comprises a first coupling structure 14, such as two or more nipples 14 projecting upwardly, i.e. forming abutments on the tongue element's 15 upper surface 15a. These nipples 14 are used for maintaining the connector 11 in the pocket 30 of the mask body as explained below.

Furthermore, the lateral arms 12a, 12b of the frame have respectively a first axis (AA) and a second axis (BB), said first axis (AA) and said second axis (BB) each forming an angle (α) of between 95° and 150°, preferably of between 120° and 140°, with the third axis (CC) as shown in Figure 4.

Accordingly, the head part 11a, 12a, 12b of the frame has a trapezoidal or quasi-trapezoidal shape or profile that matches the internal contours or shape of the pocket 30 of the mask body 1. In other words, the lateral inner walls of each pocket 30 are not parallel but instead conform to the corresponding angles of the portion of the frame that will be inserted into the pocket 30 thereby creating a kind of large trapezoidal-shaped tunnel that facilitates the insertion of the connector element 11 into the pocket 30.

When the connector element 11 is inserted into the pocket 30 of the mask, as illustrated in Figure 2, the tongue element 15 can be deformed, i.e. bent, by the insertion force and/or by digital pressure exerted by the user on the actuating zone 16, acting as a quick-release button, that is situated at the free end 15c of the tongue element 15 so that the connector element 11 penetrates into the pocket 30 and the nipples 14 cooperate with second coupling structures 31, such as lodgings, arranged in the inner wall of the pocket 30. Once, the nipples 14 are inserted into the lodgings 31, the connector element 11 is locked into the pocket 30.

As shown in Figure 3, the head part 11a of the connector 11 comprises the front portion 11a and the first portion 22 of the lateral arms 12a, 12b of the frame, that form together, respectively, the more narrow base and the two sides of a trapezoidal or quasi-trapezoidal structure. The portion 11b and the second portion 23 of the lateral arms 12a, 12b of the frame form together three sides of a rectangular or quasi-rectangular structure. The trapezoidal or quasi-trapezoidal structure of the head part 11a of the connector 11 allows an efficient, easy and quick coupling of the connector 11 in the pocket 30 of the mask body 1. An optimal relation between sizes A and B as shown in Figure 3 that allows for easy coupling of the connector element 11 to the pocket 30 is about 1.5: 1.

The straps 19 of the headgear 9 are preferably attached to the mask body 1 by the rear element 11b of the connector element 11. However, in other embodiments (not shown), the rear element 11b of the connector element 11 can have a different shape and can for example be conformed to or held on by one or more hooks, buckles or slots, for attaching the straps 19.

Preferably, the frame 11a, 11b, 12a, 12 b and the tongue element 15 are molded into one piece and made of polymer material, in particular in plastic material, such as PC, PP, ABS, nylon or PS as mentioned here above.

For releasing the connector element 11 when it has been inserted and is being held in the pocket 30 of the mask, for example when the user desires to take off the mask, the user has only to apply a digital pressure on the actuating zone 16 that is situated at the free end of the tongue element 15 so that the tongue element 15 which is made of a resilient material bends, thus allowing for the nipples to be disengaged from the second coupling structures 31. This disengagement allows for the withdrawal of the connector element 11 which was held in place by the nipples and the second coupling structures 31 being engaged.

The actuating zone acts as a quick-release button. In other words, under the pressure exerted on the flexible tongue element 15, the tongue 15 is bent and the nipples 14 come out of the lodgings 31 arranged in the wall of the pocket 30. Once, the nipples 14 are out of the lodgings 31, the connector element 11 is unlocked and can be extracted from the pocket 30. Thus, the mask can be easily removed from the patient's face.

The facial mask of the present invention can be used in a method for treatment of a respiratory disorder or condition, for example, in non-invasive positive pressure ventilation (NPPV) or in a continuous positive airway pressure (CPAP) therapy of sleep disordered breathing (SDB) conditions, such as, for example, obstructive sleep apnea (OSA).

## Claims

1. Connector element (11) comprising :
- a front portion (11a) of a third axis (CC) facing a rear portion (11b), and two lateral arms (12a, 12b) linking together said front and rear portions so as to form a frame (11a, 11b, 12a, 12 b),
- the frame (11a, 11b, 12a, 12b) bearing a tongue element (15) having an upper surface (15a), said tongue element (15) being made of a resilient material so as to be deformable, bendable or pivotable with respect to the third axis (CC), when a force is applied on the upper surface (15a) of said tongue element (15),
- the upper surface (15a) of the tongue element (15) bearing one or several first coupling structures (14), and
- the lateral arms (12a, 12b) each having respectively a first axis (AA) and a second axis (BB), said first axis (AA) and said second axis (BB) each forming an angle (α) of between 95° and 150° with the third axis (CC).

2. Connector element according to Claim 1, **characterized in that** the frame (11a, 11b, 12a, 12 b) and the tongue element (15) are molded into one piece.

3. Connector element according to any one of the preceding Claims, **characterized in that** the frame (11a, 11b, 12a, 12 b) and the tongue element (15) are made of a polymer material.

4. Connector element according to any one of the preceding Claims, **characterized in that** said first axis (AA) and said second axis (BB) each form an angle (α) of between 100° and 140° with the third axis (CC), preferably of between 105° and 130°.

5. Connector element according to any one of the preceding Claims, **characterized in that** one or more first coupling structures (14) are nipples.

6. Connector element according to any one of the preceding Claims, **characterized in that** the tongue element (15) has a general flat shape.

7. Connector element according to any one of the preceding Claims, **characterized in that** the tongue element (15) comprises an actuating zone (16) arranged at the free end (15c) of said tongue element (15), preferably the tongue element (15) is deformed, bends or pivots with respect to the third axis (CC), when a force is applied to the actuating zone (16) of the tongue element (15),

8. Connector element according to any one of the preceding Claims, **characterized in that** the tongue element (15) projects toward the center of the frame (11a, 11b, 12a, 12b).

9. Connector element according to any one of the preceding Claims, **characterized in that** the tongue element (15) is arranged on the front portion (11a) of the third axis (CC) of the frame.

10. Connector element according to any one of the preceding Claims, **characterized in that** the front portion (11a) and a first portion (22) of the lateral arms (12a, 12b) form the more narrow base with the two sides of a trapezoidal or quasi-trapezoidal structure.

11. Connector element according to any one of the preceding Claims, **characterized in that** the rear portion (11b) and a second portion (23) of the lateral arms (12a, 12b) form three sides of a rectangular or quasi-rectangular structure.

12. Respiratory mask, in particular a facial mask, comprising a mask body (1) and at least one pocket (30) arranged in the external wall of said mask body (1), said or each pocket (30) being sized and shaped so as to receive a connector element (11) according to any one of the preceding Claims, said or each pocket (30) comprising at least one second coupling structure (31) cooperating with at least one coupling structure (14) of the tongue element (15) of the connector element (11) for maintaining said connector element (11) in a locked position when inserted into one pocket (30) of the mask body (1).

13. Respiratory mask according to Claim 12, **characterized in that** the first coupling structures (14) arranged on the tongue element (15) are chosen from nipples and abutments, and the second coupling structures (31) are chosen from lodgings and grooves.

14. Respiratory mask according to Claim 12 or Claim 13, **characterized in that** the respiratory mask further comprises a headgear (9) with one or more straps (19), at least one of said straps (19) being attached to the rear portion (11b) of the connector element (11).

15. Respiratory mask according to any one of Claims 12 to 14, **characterized in that** the mask further comprises a forehead support (5), an internal chamber and a gas inlet orifice (3) in fluid communication with said internal chamber, the internal chamber comprising a peripheral border (8) bearing a cushion (10).

## Amended claims

### Amended claims in accordance with Rule 137(2) EPC.

**1.** Respiratory mask comprising a mask body (1) and at least one pocket (30) arranged in or forming part of the external wall of said mask body (1), each pocket (30) being sized and shaped so as to receive a connector element (11), and wherein :
- a connector element (11) comprising a tongue element (15) is inserted in each pocket (30) arranged in or forming part of the external wall of the mask body (1),
- each pocket (30) comprises at least one second coupling structure (31) cooperating with at least one coupling structure (14) of the tongue element (15) of the connector element (11) for maintaining said connector element (11) in a locked position when inserted into one pocket (30) of the mask body (1),
- the connector element (11) comprises a front portion (11a) of a third axis (CC) facing a rear portion (11b), and two lateral arms (12a, 12b) linking together said front and rear portions so as to form a frame (11a, 11b, 12a, 12b), the frame (11a, 11b, 12a, 12b) bearing the tongue element (15) having an upper surface (15a),
- said tongue element (15) is made of a resilient material so as to be deformable, bendable or pivotable with respect to the third axis (CC), when a force is applied on the upper surface (15a) of said tongue element (15),
- the upper surface (15a) of the tongue element (15) bears one or several first coupling structures (14),
- the lateral arms (12a, 12b) of the connector element (11) each have respectively a first axis (AA) and a second axis (BB), said first axis (AA) and said second axis (BB) each forming an angle (α) of between 95° and 150° with the third axis (CC),
- the front portion (11a) and the first portions (22) of the lateral arms (12a, 12b) form the more narrow base of a trapezoidal or quasi-trapezoidal structure,
- the rear portion (11b) and the second portions (23) of the lateral arms (12a, 12b) form three sides of a rectangular or quasi-rectangular structure,
- the tongue element (15) comprises an actuating zone (16) arranged at the free end (15c) of said tongue element (15) so that the tongue element (15) is deformed, bends or pivots with respect to the third axis (CC), when a force is applied by a user to the actuating zone (16) of the tongue element (15), and
- the first coupling structures (14) arranged on the tongue element (15) are chosen from nipples and abutments, and the second coupling structures (31) are chosen from lodgings and grooves, said first coupling structures (14) being arranged on the upper surface (15a) of the tongue element (15) and projecting upwardly.

**2.** Respiratory mask according to Claim 1, **characterized in that** the frame (11a, 11b, 12a, 12b) and the tongue element (15) of the connector element (11) are molded into one piece.

**3.** Respiratory mask according to any one of the preceding Claims, **characterized in that** the frame (11a, 11b, 12a, 12b) and the tongue element (15) of the connector element (11) are made of a polymer material.

**4.** Respiratory mask according to any one of the preceding Claims, **characterized in that** said first axis (AA) and said second axis (BB) of the connector element (11) each form an angle (α) of between 100° and 140° with the third axis (CC), preferably of between 105° and 130°.

**5.** Respiratory mask according to any one of the preceding Claims, **characterized in that** one or more first coupling structures (14) of the connector element (11) are nipples.

**6.** Respiratory mask according to any one of the preceding Claims, **characterized in that** the tongue element (15) of the connector element (11) has a general flat shape.

**7.** Respiratory mask according to any one of the preceding Claims, **characterized in that** the lateral inner walls of each pocket (30) form a trapezoidal-shaped tunnel.

**8.** Respiratory mask according to any one of the preceding Claims, **characterized in that** the tongue element (15) of the connector element (11) projects toward the center of the frame (11a, 11b, 12a, 12b).

**9.** Respiratory mask according to any one of the preceding Claims, **characterized in that** the tongue element (15) of the connector element (11) is arranged on the front portion (11a) of the third axis (CC) of the frame.

**10.** Respiratory mask according to any one of the preceding Claims, **characterized in that** the front portion (11a) and the rear portion (11b) have a length of between 5 mm and 50 mm.

**11.** Respiratory mask according to any one of the preceding Claims, **characterized in that** two pockets (30) are arranged in or forming part of the external wall of the mask body (1), one pocket (30) being arranged on each lateral side of the mask body (1) with respect to the central gas inlet or orifice (3), preferably each of the two pockets (30) receiving a connector element (11).

**12.** Respiratory mask according to any one of the preceding Claims, **characterized in that** it is a facial mask.

**13.** Respiratory mask according to Claim 1, **characterized in that** the pockets (30) are conformed and sized so as to have an internal shape that matches the shape of the connector element (11).

**14.** Respiratory mask according to Claim 1 or Claim 13, **characterized in that** it further comprises a headgear (9) with one or more straps (19), at least one of said straps (19) being attached to the rear portion (11b) of the connector element (11).

**15.** Respiratory mask according to any one of Claims 12 to 14, **characterized in that** the mask further comprises a forehead support (5), an internal chamber and a gas inlet orifice (3) in fluid communication with said internal chamber, the internal chamber comprising a peripheral border (8) bearing a cushion (10).
